# EUROPEAN PATENT SPECIFICATION

(11) **EP 1 633 334 B1**
(45) Date of publication and mention of the grant of the patent: **15.11.2006**
(21) Application number: 04776913.8
(22) Date of filing: 14.06.2004
(51) Int. Cl.: A61K 31/4415, A61K 31/455, A61K 31/4406, A61K 31/522, A61K 31/4433, A61K 47/10, A61P 25/20

(54) **TOPICAL ADMINISTRATION OF VASOACTIVE VITAMIN B3 COMPOUNDS FOR PROMOTING SLEEP**
TOPISCHE VERABREICHUNG VASOAKTIVER VITAMIN B3 VERBINDUNGEN ZUR SCHLAFFÖRDERUNG
ADMINISTRATION TOPIQUE DE COMPOSÉS DE VITAMINE B3 VASOACTIFS POUR FAVORISER LE SOMMEIL

(30) Priority: 13.06.2003 US 461701
(43) Date of publication of application: 15.03.2006
(73) Proprietor: THE PROCTER & GAMBLE COMPANY, Cincinnati, Ohio 45202 (US)
(72) Inventor: EBEL, James. Patrick, Lebanon, Ohio 45036 (US)
(74) Representative: Wilding, Richard Alan
(86) International application number: PCT/US2004/019958
(87) International publication number: WO 2005/002554

(56) References cited:
- WO-A-00/07556
- DE-A- 1 816 097
- DE-A- 2 841 170
- GB-A- 1 510 583
- US-A- 4 968 684
- US-A- 5 360 821
- US-A- 5 496 560
- US-A1- 2004 054 337
- BERRAZUETA JOSE RAMON ET AL: "Successful treatment of shoulder pain syndrome due to supraspinatus tendinitis with transdermal nitroglycerin nitroglycerin. A double blind study" PAIN, vol. 66, no. 1, 1996, pages 63-67, XP000912040 ISSN: 0304-3959
- KRAUCHI K ET AL: "Functional link between distal vasodilation and sleep-onset latency?" AMERICAN JOURNAL OF PHYSIOLOGY - REGULATORY INTEGRATIVE AND COMPARATIVE PHYSIOLOGY 2000 UNITED STATES, vol. 278, no. 3 47-3, 2000, pages R741-R748, XP008037952 ISSN: 0363-6119
- BAKER M A ET AL: "VARIABILITY OF SKIN TEMPERATURE IN THE WAKING MONKEY" AMERICAN JOURNAL OF PHYSIOLOGY, vol. 230, no. 2, 1976, pages 449-455, XP008038037 ISSN: 0002-9513
- REIMUND E: "SLEEP DEPRIVATION-INDUCED DERMATITIS FURTHER SUPPORT OF NICOTINIC ACID DEPLETION IN SLEEP DEPRIVATION" MEDICAL HYPOTHESES, vol. 36, no. 4, 1991, pages 371-373, XP008038034 ISSN: 0306-9877
- SALIN-PASCUAL R J ET AL: "Effects of transdermal nicotine on mood and sleep in nonsmoking major depressed patients" PSYCHOPHARMACOLOGY, vol. 121, no. 4, 1995, pages 476-479, XP008038029 ISSN: 0033-3158
- AGNEW L.: "Dorland's illustrated medical dictionary 24th Edition" 1967, W. B. SAUNDERS COMPANY , PHILADELPHIA, US , XP002304471 page 1667, column 1, paragraph 23
- PARFITT K.: "Martindale The Complete Drug Reference, Thirty-second Edition" 1999, PHARMACEUTICAL PRESS , LONDON , XP002304472 page 828, column 2, paragraph 2 - paragraph 5

## Description

### FIELD OF THE INVENTION

The present invention is directed to the promotion of sleep using vitamin B3 compounds that are topically applied. In particular, the present invention is directed to the provision of sleep benefits by topically administering compositions that comprise non-hypnotic, vasoactive vitamin B3 compounds.

### BACKGROUND OF THE INVENTION

Sleep disorders can be described to involve symptoms of the inability to fall asleep, interrupted sleep, insomnia, lack of restfulness, on and off sleep, and the tired, sluggish, or exhaustive feeling due to poor sleep and/or the lack of sleep. In order to alleviate symptoms related to sleep disorders, there exist products such as sleeping pills and liquid formulations comprising a sleep agent. Sleeping pills and liquid sleep formulations have been shown to provide some benefits to aid in the quality of normal sleep patterns.

Although the use of sleeping pills and liquid sleep formulations have proven to be beneficial in alleviating sleep disorders, some sleeping pills and liquid sleep formulations can result in residual drowsiness, lethargy, hangover, tired, sluggish, and/or exhaustive feelings during wake hours. It is believed that these after-sleep or wake hours feelings are contributed to narcotic and/or hypnotic sleep agents that are contained in the sleeping pills or liquid sleep formulations. It has been shown that the incorporation of narcotic and/or hypnotic sleep agents can result in habituation, dependence, or addiction in addition to tolerance or withdrawal symptoms that can be associated with the sleeping pills or liquid sleep formulations containing such sleep agents.

For example, a known sleeping pill such as melatonin has been shown to provide for normal sleep patterns. Melatonin can be administered orally, intranasally, and/or topically to treat symptoms of sleep disorders. Melatonin, however, can be classified as a hypnotic sleep aid, thus the administration of melatonin can result in unwanted effects of feeling tired or sluggish during awakening hours.

Another example of sleeping aids that can provide for sleep benefits includes agents such as diphenhydramine and the benzodiazepenes. Diphenhydramine, however, is known as a sedative that has been shown to not only provide for effective treatment of symptoms related to sleep, but the administration of diphenhydramine can also result in a tired or sluggish feeling during awakening hours. The benzodiazepenes have been shown to provide dependency and addictive properties.

Therefore, the need exists for promoting sleep by the administration of a sleeping aid that is non-hypnotic, and that provides for high quality sleep behaviors. It has been found that vitamin B3 compounds can be administered topically to provide quality sleep patterns with minimal perceived unwanted effects during wake hours.

The incorporation of non-hypnotic vitamin B3 compounds, a specific example of which includes the nicotinates, into pharmaceutical and cosmetic compositions is known. It has not, however, been demonstrated that vitamin B3 compounds can be topically applied to provide for quality sleep benefits.

In fact, the topical application of sleep agents identified in prior disclosures includes the topical application of narcotic and hypnotic compounds. For example, U.S. Patent 5,508,039 describes the transdermal administration of melatonin. WO 97/12612 discloses the administration of melatonin in combination with analgesic agents to relieve pain and induce sleep. U.S. Patent 4,968,684 and EP 285,219 disclose a method of treating sleep disorders by using N-aryl-piperazinealkanamide derivatives.

GB 1 510 583 reports that methylxanthine antagonists given orally, can antagonise the sleep disturbing characteristics of methylxanthines, such as caffeine.

It has been found that topical application of compositions comprising a vitamin B3 compound is highly effective in promoting quality sleep. The topical administration of vitamin B3 compounds can result in the dilation of the blood vessels creating a phenomenon known as vasodilation. Accordingly, the promotion of sleep by topically administering compositions comprising a vitamin B3 compound that causes dilation of blood vessels (i.e., vasodilation) is beneficial in improving the quality of sleep.

### SUMMARY OF THE INVENTION

The present invention is directed to the promotion of sleep by topically applying to mammalian skin a composition comprising a non-hypnotic vasoactive vitamin B3 compound.

It has been found that the quality of sleep can be improved by topically applying compositions comprising a non-hypnotic vitamin B3 compound, wherein the vitamin B3 compound provides for dilation of the blood vessels to result in normal sleep patterns. The application includes the topical application of the compositions to any area of the skin including the head, face, arms, forearms, elbows, hands, stomach, buttocks, legs, knees, ankles, and feet.

### DETAILED DESCRIPTION OF THE INVENTION

The present invention is directed to the promotion of sleep by the topical application of compositions comprising a vitamin B3 compound. The vitamin B3 compound provides for the dilation of blood vessels to result in improved quality of sleep.

The term "sleep disorders" as used herein refers to the onset, duration, and/or after effect of the lack of sleep. In other words, "sleep disorders" refer to the lack of ability to fall asleep, interrupted sleep, insomnia, lack of restfulness, on and off sleep, and the tired, sluggish, or exhaustive feeling due to the lack of sleep. The term "sleep" is typically referred to as the state of rest that occurs periodically and that is characterized by relative physical nervous inactivity, lessened responsiveness, unconsciousness, and typical brain wave patterns as measured using electroencephalography.

The term "hypnotic" as used herein refers to actives and drugs that induce sleep through direct central nervous system (CNS) depression, or other related CNS activity. Examples of hypnotics include the imidazopyridines, pyrazolopyrimidines, benzodiazepines, barbiturates, antianxiety drugs, opiods, sedating antihistamines, antipsychotics, tranquilizers, antiiolytics, and antidepressants. The term "non-hypnotic" as used herein refers to substances, compounds and materials that do not have direct CNS depressant effects, or other related CNS activity.

The term "vasoactive" as used herein refers to compounds and materials that can cause a dilation of blood vessels. It is understood that the term "vasoactive" includes constriction and dilation of blood vessels, however, as used herein the term "vasoactive" refers to the dilation of blood vessels.

The present invention includes the topical application of compositions that can comprise, consist of, or consist essentially of the elements described herein, as well as any of the additional or optional ingredients or components described herein.

### Vasoactive Vitamin B3 compound

The present invention is directed to the promotion of sleep by the topical application of compositions that comprise a vitamin B3 compound. The vitamin B3 compound is non-hypnotic, and provides for the dilation of blood vessels to result in improved quality of sleep without any perceived negative after-sleep feelings of sedation, drowsiness, lethargy, hangover, tiredness and/or exhaustion. The non-hypnotic, vasoactive vitamin B3 compound also provides for compositions that are not potentially habit forming, low tolerant, dependent, or can cause withdrawal symptoms.

The vitamin B3 compound can be included in the compositions as an individual vitamin B3 compound or as a combination of vitamin B3 compounds, wherein the total concentration of the vitamin B3 compound typically ranges from 0.001 % to 25%, preferably from 0.01 % to 10%, more preferably from 0.05% to 5%, by weight of the composition.

Specific vitamin B3 compounds which are suitable for use as a preferred vitamin B3 compound herein include the nicotinates. Specific nicotinates include benzyl nicotinate, inositol hexanicotinate, nicotinic acid, nicotinyl alcohol, xanthine nicotinate, methyl nicotinate, ethyl nicotinate, propyl nicotinate, isopropyl nicotinate, butyl nicotinate, isoamyl nicotinate, hexyl nicotinate, phenyl nicotinate, guaiacyl nicotinate, caffeine nicotinate, xanthinol nicotinate, nicametate citrate, nicotinamide, nicotinuric acid, nicotinyl hydroxamate, tocopheryl nicotinate, and mixtures thereof.

### Compositions

The use of the present invention includes the topical application of the vitamin B3 compounds described hereinabove. The vitamin B3 compound is typically incorporated into compositions that can be topically applied. The compositions include any known or otherwise effective composition that can be topically applied to mammalian skin. The terms "topical application" and "topically applied" are used interchangeably herein to refer to the application onto the outer layer of mammalian skin, which include application by rubbing onto the skin, brushing, painting, wiping and stroking.

Examples of compositions that can be topically applied include those known or otherwise effective compositions suitable for use in beauty, pharmaceutical, cosmetic, and skin care technologies. Specific examples of such compositions include moisturizers, lotions, astringents, facial and skin cleansers, body oils, ointments, gels and creams. These compositions typically comprise a carrier system that contains the vitamin B3 compound.

The carrier system of the compositions defined herein include aqueous systems, nonaqueous systems, emulsion systems, and mixtures thereof. Specific examples of solvents that can be included in these carrier systems include oils, alcohols, silicones, glycols, and mixtures thereof. Specific examples of suitable emulsion systems include oil-in-water emulsions, water-in-oil emulsions, oil-in-water-in-silicone emulsions, and mixtures thereof.

The compositions typically comprise the carrier system at concentrations ranging from 75% to 99.999%, preferably from 90% to 99.99%, more prefereably from 95% to 99.95%, by weight of the composition.

In addition to the carrier system, the compositions can comprise optional components that are known or otherwise effective for use in beauty, pharmaceutical, cosmetic, and skin care compositions, provided that the optional components are physically and chemically compatible with the vitamin B3 compound defined herein or do not otherwise unduly impair product stability, aesthetics, or performance. Optional components suitable for use herein include materials such as pH adjusting agents, preservatives, humectants, moisturizers, fragrances, surfactants, skin penetration enhancers, and so forth. The optional components are typically included at concentrations ranging from 0.01% to 50%, preferably from 0.1% to 10%, by weight of the composition.

### Use

The present invention is directed to the promotion of sleep by topically applying onto the skin of mammalians compositions that comprise a vitamin B3 compound defined herein. Typically a safe and effective amount of the composition is to be applied to the skin. In this context, the term "safe and effective amount" refers to an amount with provides a therapeutic benefit with minimal or no adverse reactions.

As referred to herein, the promotion of sleep includes any known or otherwise effective way of promoting sleep patterns and behaviors that provide for the onset and/or duration of sleep, which includes the patterns and behaviors that are exhibited prior to falling asleep. Examples of such patterns and behaviors include the calm and/or relaxed feeling prior to falling asleep, uninterrupted sleep, restful sleep, drowsiness, slumber sleep, dozing, nodding, and mixtures thereof.

To promote sleep, compositions that comprise a vitamin B3 compound defined herein are to be topically applied onto mammalian skin. In general, from 0.1 milligram of composition/square centimeter of skin (mg/cm²) to 10 mg/cm² is to be topically applied to result in from 0.1 mg to 100 mg, preferably from 1mg to 10 mg of vitamin B3 compound being applied onto the skin.

The compositions can be topically applied to any area of mammalian skin, including areas of the head, face, arms, forearms, elbows, hands, stomach, buttocks, legs, knees, ankles, feet, and mixtures thereof. The same or different amounts of the composition can be applied, provided that the total amount of composition being applied onto the skin does not exceed 10 mg/cm² for a single application process, wherein a single application process includes one or more applications onto one or more areas of mammalian skin.

The compositions can be applied using a single application or multiple application processes prior to falling asleep to improve the quality of sleep. The application processes can vary with the area of the skin onto which a composition is applied, for example an application process can involve the application of a composition onto the arm one or more times to result in improved quality of sleep, or alternatively one or more application processes can involve application onto the hands and/or legs one or more times to improve the quality of sleep. It is believed that the area of skin onto which a composition is applied will influence the amount of change in skin and/or core body temperature to result in a higher or lower propensity to fall asleep in addition to improved sleep patterns.

The compositions can be applied onto the skin using any known or otherwise effective application technique including the techniques of rubbing, brushing, painting, wiping, and stroking a composition onto the skin. A highly effective method of promoting sleep has been developed by applying a composition onto the skin one or more times prior to bedtime, wherein the composition comprises a vitamin B3 compound.

### EXAMPLES

The following examples further describe and demonstrate embodiments within the scope of the present invention. The examples are given solely for the purpose of illustration. All exemplified concentrations are weight-weight percents, unless otherwise specified.

Compositions that can be topically applied onto the skin to improve sleep quality are exemplified hereinbelow. The compositions comprise a vitamin B3 compound that is highly effective in providing for improved sleep benefits. The compositions can be applied onto one or more areas of the skin one or more times prior to bedtime to result in improved sleep quality.

### EXAMPLE 1

Below is an example of a gel composition suitable for use in the present invention. The gel composition is formed by combining and mixing the ingredients using known conventional gel forming processes. The gel is to be applied onto the outer skin surface of the hands and feet prior to bedtime.

| **Ingredient** | **Weight %** |
|---|---|
| Methyl nicotinate | 0.10 |
| SD alcohol 40 | 20.0 |
| Carbopol EDT 2001 | 1.00 |
| Sodium hydroxide, 50% | 0.60 |
| Deionized water | q.s to 100 |

### EXAMPLE 2

Below is an example of a skin cream suitable for use in the present invention. The skin cream is formed by combining and mixing the ingredients using known conventional processes for formulating skin creams. The skin cream is to be applied to skin areas of the lower legs and feet prior to bedtime.

| **Ingredient** | **Weight %** |
|---|---|
| Benzyl nicotinate | 0.20 |
| Carbopol 980 | 0.50 |
| Hydroxyethylcellulose | 0.50 |
| SD alcohol 40 | 20.0 |
| DC 556 silicone fluid | 2.00 |
| Witch hazel distillate | 0.20 |
| Sodium hydroxide, 50% | 1.00 |
| Fragrance | 0.10 |
| Deionized water | q.s. to 100 |

### EXAMPLE 3

Below is an example of a skin cream suitable for use in the present invention. The skin cream is formed by combining and mixing the ingredients using known conventional processes for formulating skin creams. The skin cream is to be applied to skin areas of the forearms and hands prior to bedtime.

| **Ingredient** | **Weight %** |
|---|---|
| Inositol hexanicotinate | 0.50 |
| Glycerine | 7.00 |
| Isododecane | 3.00 |
| Polyacrylamide, C13-C14 isoparaffin, Laureth-7 | 2.50 |
| Dimethicone, Dimethiconol | 2.00 |
| Isopropyl isostearate | 1.33 |
| Sorbitan monostearate, Sucrocoate | 1.00 |
| Cetyl alcohol | 0.72 |
| Tocopherol acetate | 0.50 |
| Panthenol | 0.50 |
| Stearyl alcohol | 0.50 |
| Deionized water | q.s. to 100 |

## Claims

1. The use of a vitamin B3 compound in the manufacture of a medicament for promoting sleep by topical application of the medicament to mammalian skin.

2. The use of claim 1 wherein the vitamin B3 compound is selected from the group consisting of benzyl nicotinate, inositol hexanicotinate, nicotinic acid, nicotinyl alcohol, xanthine nicotinate, methyl nicotinate, ethyl nicotinate, propyl nicotinate, isopropyl nicotinate, butyl nicotinate, isoamyl nicotinate, hexyl nicotinate, phenyl nicotinate, guaiacyl nicotinate, caffeine nicotinate, xanthinol nicotinate, nicametate citrate, nicotinamide, nicotinuric acid, nicotinyl hydroxamate, tocopheryl nicotinate, or mixtures thereof.

3. The use according to claim 1 or claim 2 wherein from 0.1 mg/cm² to 10 mg/cm² of the medicament is to be applied topically to the mammalian skin.

4. The use according to any one of the preceding claims wherein the medicament is to be applied to areas of the mammalian skin selected from the group consisting of head, face, arms, forearms, elbows, hands, stomach, buttocks, legs, knees, ankles, feet, or mixtures thereof.

## Patentansprüche

1. Verwendung einer Vitamin-B3-Verbindung bei der Herstellung eines Medikaments zur Förderung von Schlaf durch äußerliches Auftragen des Medikaments auf die Haut von Säugern.

2. Verwendung nach Anspruch 1, wobei die Vitamin-B3-Verbindung ausgewählt ist aus der Gruppe, bestehend aus Benzylnicotinat, Inosithexanicotinat, Nicotinsäure, Nicotinylalkohol, Xanthinnicotinat, Methylnicotinat, Ethylnicotinat, Propylnicotinat, Isopropylnicotinat, Butylnicotinat, Isoamylnicotinat, Hexylnicotinat, Phenylnicotinat, Guajacylnicotinat, Koffeinnicotinat, Xanthinolnicotinat, Nicametatcitrat, Nicotinamid, Nicotinursäure, Nicotinylhydroxamat, Tocopherylnicotinat oder Mischungen davon.

3. Verwendung nach Anspruch 1 oder Anspruch 2, wobei von 0,1 mg/cm² bis 10 mg/cm² der Zusammensetzung äußerlich auf die Haut von Säugern aufzutragen sind.

4. Verwendung nach einem der vorstehenden Ansprüche, wobei das Medikament auf Bereiche der Haut von Säugern, ausgewählt aus der Gruppe, bestehend aus Kopf, Gesicht, Armen, Unterarmen, Ellenbögen, Händen, Bauch, Gesäß, Beinen, Knien, Knöcheln, Füßen und Mischungen davon aufzutragen ist.

## Revendications

1. Utilisation d'un composé de vitamine B3 dans la fabrication d'un médicament pour favoriser le sommeil par application locale du médicament sur la peau mammalienne.

2. Utilisation selon la revendication 1, dans laquelle le composé de vitamine B3 est choisi dans le groupe constitué de nicotinate de benzyle, hexanicotinate d'inositol, acide nicotinique, alcool nicotinylique, nicotinate de xanthine, nicotinate de méthyle, nicotinate d'éthyle, nicotinate de propyle, nicotinate d'isopropyle, nicotinate de butyle, nicotinate d'isoamyle, nicotinate d'hexyle, nicotinate de phényle, nicotinate de guaiacyle, nicotinate de caféine, nicotinate de xanthinol, citrate de nicamétate, nicotinamide, acide nicotinurique, hydroxamate de nicotinyle, nicotinate de tocophéryle, ou leurs mélanges.

3. Utilisation selon la revendication 1 ou la revendication 2, dans laquelle de 0,1 mg/cm² à 10 mg/cm² de la composition doit s'appliquer localement sur la peau mammalienne.

4. Utilisation selon l'une quelconque des revendications précédentes, dans laquelle le médicament doit s'appliquer sur des zones de la peau mammalienne choisies dans le groupe constitué de la tête, du visage, des bras, des avant-bras, des coudes, des mains, du ventre, des fesses, des jambes, des genoux, des chevilles, des pieds, et leurs mélanges.
